# EUROPÄISCHE PATENTANMELDUNG

(11) **EP 4 403 617 A1**
(43) Veröffentlichungstag der Anmeldung: **24.07.2024**
(21) Anmeldenummer: 23152324.2
(22) Anmeldetag: 18.01.2023
(51) Int. Cl.: C12M 1/00

(54) **VORRICHTUNG UND VERFAHREN ZUR AUFZUCHT VON ALGEN IN EINEM GEWÄSSER**

(71) Anmelder: Yan, On-Yee, 10967 Berlin (DE); Worbes, Arthur, 38173 Erkerode/Lucklum (DE); Büttner, Bernhard, 10781 Berlin (DE); Lempp, Alexander, 24148 Kiel (DE)
(72) Erfinder: Yan, On-Yee, 10967 Berlin (DE); Worbes, Arthur, 38173 Erkerode/Lucklum (DE); Büttner, Bernhard, 10781 Berlin (DE); Lempp, Alexander, 24148 Kiel (DE)
(74) Vertreter: Heinemeyer, Karsten

(57) **Zusammenfassung**

Beschrieben werden eine Vorrichtung (1) und ein Verfahren zur Aufzucht von Algen in einem Gewässer (10). Die Vorrichtung (1) verfügt über einen Grundkörper (2), an dem ein Substratträger (3) mit einer Vegetationsfläche (4) zur Aufbringung eines Substrats (5), in dem sich Samen, Keimlinge, Sämlinge und/oder Setzlinge befinden, befestigbar oder befestigt ist, wobei der Substratträger (3) mit der Vegetationsfläche (4) zumindest zeitweise unter eine Wasseroberfläche (9) des Gewässers (10) absenkbar ist.

Die beschriebene technische Lösung zeichnet sich dadurch aus, dass eine Tarriereinheit (6) vorgesehen ist, die wenigstens mittelbar mit dem Substratträger (3) verbunden oder verbindbar ist und die über einen Schwimmkörper (7) und über eine Stelleinheit (8), durch die eine Dichte des Schwimmkörpers (7) veränderbar ist, verfügt.

## Beschreibung

Die Erfindung betrifft eine Vorrichtung sowie ein Verfahren zur Aufzucht von Algen in einem Gewässer. Die beschriebene Vorrichtung verfügt über einen Grundkörper, an dem ein Substratträger mit einer Vegetationsfläche zur Aufbringung eines Substrats, in dem sich Samen, Keimlinge, Sämlinge und/oder Setzlinge befinden, befestigbar oder befestigt ist., wobei der Substratkörper gemeinsam mit der Vegetationsfläche zumindest zeitweise gezielt unter eine Wasseroberfläche des Gewässers absenkbar ist.

Allgemein handelt es sich bei Algen um eukaryotische Lebewesen, die im Wasser leben und Photosynthese betreiben. Sie sind in vielen Lebensmitteln und Kosmetika enthalten und es wird derzeit davon ausgegangen, dass Algen einen nicht unerheblichen Beitrag zur Nahrungsmittelversorgung der wachsenden Weltbevölkerung liefern können. Algenfarmen existieren in unterschiedlichen küstennahen Gebieten auf der Erde, bevorzugt in Gezeitenrevieren, insbesondere in Lagunen oder küstennahen Flachwasserregionen, wobei die Hauptanbaugebiete derzeit noch in Asien liegen. Nichtsdestotrotz findet auch in deutschen Gewässern Algenanbau statt, hier insbesondere in den Wintermonaten.

Grundsätzlich sind aus dem Stand der Technik zwei unterschiedliche Lösungsansätze bekannt, die für die gezielte Algenaufzucht verwendet werden, nämlich einerseits der Anbau im Meer, andererseits aber auch der Anbau in speziellen Anbauvorrichtungen an Land. In diesem Zusammenhang ist aus der DE 198 35 656 A1 ein Verfahren sowie eine Vorrichtung zur Algenkultivierung bekannt, bei dem das Algenwachstum in einer feuchten Atmosphäre innerhalb eines Kultivierungsraumes erfolgt. Hierbei verfügt der Kultivierungsraum über eine Verankerungseinrichtung zur Befestigung der zu kultivierenden Algen und über eine Flüssigkeitszufuhr, durch die ein Nährmedium in Form eines Aerosols oder als Sprühstrahl in den Kultivierungsraum eingebracht wird. In der beschriebenen Anlage können die Wachstumsbedingungen für die Algen, etwa die Temperatur, das Nährmedium und die Beleuchtung bedarfsgerecht eingestellt werden. Nachteilig an dem beschriebenen System ist allerdings der vergleichsweise große apparative Aufwand, der Energie-, Wasser- sowie Platzbedarf an Land.

Im Vergleich zu den Anlagen, die für die kontrollierte Algenaufzucht an Land, beispielsweise für die kosmetische und pharmazeutische Industrie, verwendet werden, zeichnen sich Algenfarmen im Meer durch einen sehr einfachen Aufbau aus. Üblicherweise werden Algensetzlinge an langen Leinen oder Netzen, die teilweise spezielle Haltesysteme aufweisen, befestigt und gemeinsam mit diesen im Meer ausgebracht. Nach einigen Monaten werden die Leinen oder Netze mit den zwischenzeitlich gewachsenen Algen von Schiffen aus eingeholt und die Algen mit Hilfe von geeigneten Maschinen von den Leinen oder Netzen abgeschnitten. Problematisch an diesem Verfahren zur Algenaufzucht ist vor allem, dass die Leinen oder Netze Kunststoffmaterial aufweisen, teilweise nur einmal verwendet werden und in den Meeren eine große Anzahl von Leinen oder Netzen, sogenannte Geisternetze, verbleibt, sodass die Umwelt hierdurch stark belastet wird. Ebenso ist nicht auszuschließen, dass sich während der Ernte der Algen, also wenn diese von den Leinen oder Netzen abgeschnitten werden, Kunststoffe in die geernteten Algen eingetragen werden, was letztendlich zu einer ungewünschten Verschmutzung dieses Naturprodukts führt. Im Weiteren ist es bei dieser Form des Algenanbaus nicht oder nur sehr schwer möglich, während der Wachstumsphase die Position der zu kultivierenden Algen zu verändern, beispielsweise um bessere Wachstumsbedingungen erreichen zu können. Darüber hinaus ist zu beachten, dass viele Algensorten nicht an Leinen gezüchtet werden können, da die Oberfläche der Leinen für die Aufzucht dieser Algen ungeeignet ist.

Ausgehend von den aus dem Stand der Technik bekannten Lösungen zur agrarindustriellen Algenaufzucht sowie den zuvor geschilderten Problemen liegt der Erfindung die Aufgabe zu Grunde, eine Vorrichtung sowie ein Verfahren zur Algenaufzucht anzugeben, mit dem auf vergleichsweise einfache und effektive Weise eine nachhaltige Aufzucht von Algen ermöglicht wird. Hierbei ist es einerseits wünschenswert, dass die Positionierung der für die Kultivierung von Algen erforderlichen Samen, Keimlinge, Sämlinge und/oder Setzlinge sowie der daraus hervorgehenden Algen während ihrer Wachstumsphase an die jeweils in offenen Gewässern variierenden Wachstumsbedingungen anpassbar ist. Insbesondere sollte die Anordnung der Samen, Keimlinge, Sämlinge und/oder Setzlinge sowie der daraus hervorgehenden Algen bedarfsgerecht an die in verschiedenen Wassertiefen herrschenden unterschiedlichen Temperaturen, Nährstoffgehalte, Salzgehalte, Strömungen und die auf die Algen auftreffende Sonnenstrahlung anpassbar sein.

Weiterhin sollte für die Lösung der der Erfindung zugrunde liegenden Aufgabe berücksichtigt werden, dass die anzugebende Vorrichtung und das anzugebende auch mit Materialien realisierbar sind, die sich durch ihre Umweltverträglichkeit auszeichnen und bevorzugt sortenrein, lebensmittelecht und auch über längere Zeiträume wasserbeständig sind. Insbesondere bei Verwendung von Kunststoffen sollten diese in Wasser langlebig, wiederverwendbar und/oder recyclingfähig sein sowie der Eintrag von Kunststoffpartikeln in Gewässer zumindest minimiert werden. Außerdem sollte eine technische Lösung für eine agroindustrielle Algenaufzucht bereitgestellt werden, deren Energiebedarf, Wasserverbrauch und Umweltbeeinflussung gegenüber den bekannten Systemen zumindest minimiert wird.

Die zuvor beschriebene Aufgabe wird mit einer Vorrichtung gemäß Anspruch 1 sowie einem Verfahren nach Anspruch 16 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert Hierbei gelten technische Merkmale, die im Zusammenhang mit der Beschreibung einer Vorrichtung oder einem Verfahren offenbart sind, wechselseitig auch für den jeweils anderen Gegenstand als offenbart. Insofern gelten sowohl die Umsetzung gegenständlicher Merkmale der beschriebenen Vorrichtung durch entsprechende Verfahrensschritte als auch die Realisierung der Verfahrensschritte durch geeignet ausgebildete Vorrichtungen als mitoffenbart.

Der Erfindung betrifft eine Vorrichtung zur Aufzucht von Algen in einem Gewässer mit einem Grundkörper, an dem ein Substratträger mit einer Vegetationsfläche zur Aufbringung eines Substrats, in dem sich Samen, Keimlinge, Sämlinge und/oder Setzlinge befinden, befestigbar oder befestigt ist, wobei der Substratträger mit der Vegetationsfläche zumindest zeitweise unter eine Wasseroberfläche des Gewässers absenkbar ist. Selbstverständlich schließt diese Lösung mit ein, dass auch eine Mehrzahl von Substratträgern, also wenigstens zwei Substratträger, vorgesehen sein können. Die Erfindung zeichnet sich dadurch aus, dass eine Tarriereinheit vorgesehen ist, die wenigstens mittelbar mit dem Substratträger verbunden oder verbindbar ist und die über einen Schwimmkörper und über eine Stelleinheit, durch die eine Dichte veränderbar ist, verfügt. Generell ist es hierbei denkbar, dass zur bedarfsgerechten Variation der Dichte des Schwimmkörpers die Masse und/oder das Volumen des Schwimmkörpers durch Einsatz der Stelleinheit verändert wird.

Mit Hilfe der Tarriereinheit ist somit möglich, derart gezielt bzw. gesteuert auf den Substratträger einzuwirken, dass eine Tiefe, in der der Substratträger angeordnet wird, bedarfsgerecht veränderbar ist. Auf vorteilhafte Weise ist somit eine Steuerung vorgesehen, mit der wenigstens ein Steuersignal erzeugbar ist, um eine Aktion der Stelleinheit, beispielsweise die Bewegung eines Stellmotors, einer Pumpe, einer Verdichtereinheit und/oder wenigstens eines Stellorgans, wie etwa eines Ventils, zu bewirken. Von besonderem Vorteil ist es darüber hinaus, wenn das Steuersignal auf der Basis wenigstens eines Messwerts eines für das Wachstum der Algen relevanten Parameters erzeugt wird, sodass ein geregelter Prozess zur Einstellung der Tiefe, in der sich der Substratträger unterhalb der Wasseroberfläche des Gewässers befindet, realisierbar ist.

Wesentlich für die Erfindung ist somit das Vorsehen einer Tarriereinheit mit einer Stelleinheit und einem geeigneten Schwimmkörper, wobei die gezielt verstellbar ist, sodass die erfindungsgemäße Vorrichtung, insbesondere der Substratträger, bedarfsgerecht in Richtung des Gewässeruntergrundes oder der Gewässeroberfläche bewegt oder aber in einer bestimmten Tiefe unterhalb der Wasseroberfläche gehalten werden kann. Die Dichte des Schwimmkörpers lässt sich somit derart verändern, dass hierdurch auch das Verhältnis zwischen der Auftriebskraft, die unterhalb der Oberfläche des Gewässers auf die erfindungsgemäße Vorrichtung wirkt, und der auf diese einwirkenden Schwerkraft, gezielt variierbar ist.

Die erfindungsgemäße technische Lösung ermöglicht es somit, den Substratträger zielgerichtet in eine Solltiefe unterhalb der Wasseroberfläche zu verbringen, in der die Wachstumsbedingungen zum jeweiligen Zeitpunkt zumindest annähernd optimal für die zu kultivierenden Algen sind und ihn bei Bedarf wieder in Richtung der Oberfläche des Gewässers zu bewegen. In diesem Zusammenhang ist es auf vorteilhafte Weise denkbar, dass die Solltiefe nicht konstant ist, sondern insbesondere auf sich unterhalb der Wasseroberfläche des Gewässers ändernde Wachstumsbedingungen für die zu kultivierenden Algen bedarfsgerecht angepasst wird. Gemäß der Erfindung lässt sich der Grundkörper der Vorrichtung mit dem daran befestigten Substratträger, und damit die zu kultivierenden Algen, durch gezielte Ansteuerung der Tarriereinheit, insbesondere der Stelleinheit, in die jeweils gewünschte Wassertiefe verbringen. In Bezug auf den Substratträger wird angemerkt, dass es sich um einen, bevorzugt aber um eine Mehrzahl von Substratträgern handeln kann, der oder die zumindest mittelbar am Grundkörper zu befestigen sind. Im Übrigen ist es denkbar, dass ein Substratträger aus einer Mehrzahl von Substratträgerelementen besteht. Auch wenn im Folgenden von dem oder einem Substratträger gesprochen wird, sind hiermit die zuvor erwähnten unterschiedlichen Ausführungsformen mitumfasst. Auf vorteilhafte Weise lässt sich der Substratträger oder eine Mehrzahl von Substratträgern lösbar fest und/oder auswechselbar, bevorzugt ohne dass hierfür ein Werkzeug benötigt wird, also werkzeugfrei, am Grundkörper befestigen. In diesem Zusammenhang ist es denkbar, dass für eine Befestigung des Substratträgers am Grundkörper geeignete Befestigungselemente, wie etwa Schrauben und/oder Rastelemente vorgesehen sind.

Gemäß einer besonderen Weiterbildung der Erfindung ist vorgesehen, dass die Tarriereinheit Teil des Grundkörpers ist oder den Grundkörper bildet. Auf diese Weise kann eine besonders kompakte Einheit geschaffen werden, bei der die Tarriereinheit, die beispielsweise einen Schwimmkörper mit einem Hohlraum, der durch die Stelleinheit mit unterschiedlichen Medien füllbar ist, aufweist, in unmittelbarer Nähe des Substratträgers angeordnet ist.

In einer speziellen Weiterbildung der Erfindung ist vorgesehen, dass die Vegetationsfläche des Substratträgers unterhalb eines Abdeckelements angeordnet ist, das eine Mehrzahl von Öffnungen aufweist, durch Wasser in eine zwischen dem Abdeckelement und der Vegetationsfläche angeordneten Zwischenraum einströmen kann. Vorzugsweise verfügt das Abdeckelement über geeignete Befestigungsmittel, sodass das Abdeckelement lösbar fest oberhalb der Vegetationsfläche des Substratträgers am Substratträger befestigbar ist. Das Abdeckelement ist hierbei bevorzugt derart am Substratträger befestigt, dass dieses auf vergleichsweise einfache Weise abgenommen, daraufhin ein Substrat auf die Vegetationsfläche aufgebracht und schließlich das Abdeckelement wieder am Substratträger befestigt werden kann. Wesentlich ist jeweils, dass das Abdeckelement über geeignete Öffnungen verfügt, durch die während der Kultivierung der Algen Wasser in den Bereich der Vegetationsfläche und des Substrats eindringen kann, sodass die Algen in ausreichendem Maße mit Wasser und Nährstoffen versorgt werden. Hierbei ist es generell denkbar, dass der Substratträger und/oder das Abdeckelement eben, gekrümmt oder auf sonstige Weise geformt ist bzw. sind.

Gemäß einer besonderen Weiterbildung ist vorgesehen, dass das Abdeckelement eine Perforation, insbesondere ein Gitter und/oder eine Lochplatte aufweist. Eine entsprechende Auswahl eines geeigneten Abdeckelements wird unter Berücksichtigung der zu kultivierenden Algenart, der in einem Gewässer herrschenden Wachstumsbedingungen und/oder der jeweiligen Wachstumsphase der zu kultivierenden Algen getroffen. Während sich der Substratträger mit der Vegetationsfläche und dem hierauf angeordneten Substrat zumindest teilweise unterhalb der Wasseroberfläche eines Gewässers befindet, gelangt somit Wasser durch die Öffnungen des Gitters und/oder der Lochplatte hindurch in das Substrat, das auf diese Weise befeuchtet und mit Nährstoffen versorgt wird.

In einer weiteren speziellen Ausführungsform der Erfindung ist Vorgesehen, dass der Substratträger selbst ein Material aufweist, eine spezielle Oberfläche aufweist und/oder auf geeignete Weise strukturiert ist, sodass hierdurch die Vegetationsfläche gebildet wird und das Substrat mit den darin angeordneten Samen, Keimlingen, Sämlingen, Setzlingen und/oder Wurzeln sicher an der Vegetationsfläche des Substratträgers anhaften. So könnte der Substratträger zumindest bereichsweise eine poröse Struktur, etwa mit Öffnungen, Sacklöchern, Durchgangslöchern und/oder in Form eines Gitters aufweisen, wobei diese Struktur das Substrat zuverlässig, insbesondere in seinem Inneren, hält. Vorzugsweise weist der Substratträger daher Tonmaterial auf und/oder die Oberfläche des Substratträgers ist wenigstens abschnittsweise aufgeraut. Sofern der Substratträger derart gestaltet ist, dass das Substrat fest an und/oder in diesem haftet, hat dies insbesondere den Vorteil, dass auf ein Abdeckelement verzichtet werden kann, aber nicht verzichtet werden muss.

Im Weiteren ist es von besonderem Vorteil, wenn der Substratträger und/oder das Abdeckelement aus einem biologisch abbaubaren oder zumindest auf besondere Weise umweltverträglichen, nicht gewässergefährdenden Material hergestellt ist. Dies ist von besonderer Bedeutung, da bei einem längeren Aufenthalt der Vorrichtung zur Algenaufzucht in einem Gewässer nicht gänzlich ausgeschlossen werden kann, dass sich Teile des Materials, aus dem der Substratträger und/oder das Abdeckelement gebildet sind, zumindest teilweise ablösen und in das Gewässer gelangen. Dies stellt beispielsweise bei dem gemäß den bekannten Vorrichtung aus dem Stand der Technik genutzten Kunststoffleinen und Kunststoffnetzen ein nicht unerhebliches Problem für die Umwelt und somit eine besondere Umweltbelastung dar. Denkbar ist in diesem Zusammenhang, dass der Substratträger und/oder das Abdeckelement aus einem anderen Naturstoff hergestellt ist, der über besondere Widerstandsfähigkeit gegenüber Wasser verfügt und vergleichsweise lange im Wasser gelassen werden kann, ohne dass es zu Beschädigungen des entsprechenden Materials kommt. Vorzugsweise sind der Substratträger und/oder das Abdeckelement aus Holz.

Im Weiteren ist es von besonderem Vorteil, wenn auf der Vegetationsfläche wenigstens bereichsweise ein Schwammmaterial angeordnet ist und/oder das Substrat wenigstens teilweise ein Schwammmaterial aufweist. In diesem Zusammenhang ist es denkbar, dass zunächst Samen, Keimlinge, Sämlinge und/oder Setzlinge in einem solchen Schwammmaterial angeordnet und gemeinsam mit dem Substrat auf der Vegetationsfläche des Substratträgers angeordnet werden. Ebenso ist es denkbar, dass sich auf der Vegetationsfläche ein schwammartiges Material befindet, auf das zur Beschickung des Substratträgers ein Substrat mit Samen, Keimlingen, Sämlingen und/oder Setzlingen aufgebracht wird. Von besonderem Vorteil bei der Verwendung eines Schwammmaterials ist, dass dieses Material Wasser und/oder Nährstoffe speichern oder auch bedarfsgerecht mit Nährstoffen imprägniert werden kann, bevor der Substratträger mit dem darauf befestigten Substrat im Gewässer abgesenkt wird. In diesem Fall wird auch in Gewässern mit niedrigen Nährstoffgehalt eine gleichmäßige Versorgung der zu kultivierenden Algen mit Nährstoffen, die in diesem Fall vorzugsweise in dem Schwammmaterial gespeichert und über einen längeren Zeitraum abgegeben werden, denkbar.

Im Weiteren ist es ebenfalls denkbar, dass es sich bei einem entsprechend mit Nährstoffen imprägnierten Schwammmaterial um ein Wachstumssubstrat handelt, indem zusätzlich zu den Samen, Keimlingen, Sämlingen und/oder Setzlinge bereits spezielle Nährstoffe enthalten sind. Die jeweilige Zusammensetzung des Substrats, insbesondere die Wahl des Wachstumssubstrat und die Mischungsverhältnisse können hierbei bedarfsgerecht an das jeweilige Gewässer in dem Algen kultiviert werden sollen, und insbesondere an die Wachstumsbedingungen des Gewässers, die maßgeblich von dem Nährstoffgehalt, Sauerstoffgehalt, Salzgehalt, Nitratgehalt, der Temperatur, den Strömungsverhältnissen und/oder der Gewässeroberflächenbestrahlung, insbesondere der Sonneneinstrahlung, beeinflusst werden eingestellt werden.

In einer weiteren Ausführungsform der Erfindung ist vorgesehen, dass an dem Grundkörper wenigstens eine Aufnahme zur zerstörungsfreien Befestigung des Substratträgers und zum zerstörungsfreien Lösen des befestigten Substratträgers angeordnet sind. Gemäß dieser Ausführungsform ist es somit möglich, einen Substratträger oder eine Mehrzahl von Substratträger auf vergleichsweise einfache Weise an dem Grundkörper der Vorrichtung zur Aufzucht von Algen zu befestigen. Denkbar ist in diesem Zusammenhang, dass der Grundkörper über Aufnahmen oder einzelne Öffnungen verfügt, in die entsprechende Gegenelemente, die sich am Substratträger befinden, eingeschoben, eingesteckt, eingeschraubt, eingeklemmt und/oder eingerastet werden können, so dass der Substratträger relativ in seiner Position zum Grundkörper fixiert und auch wieder gelöst werden kann. Von besonderem Vorteil ist es in diesem Fall, wenn der oder die Substratträger an dem Grundkörper ohne Einsatz von Werkzeugen zu befestigen ist und dieser oder diese auch wieder ohne Einsatz von Werkzeug vom Grundkörper lösbar ist, beispielsweise durch Einsatz geeigneter Rastelemente, die beim Befestigen des Substratträgers an den Grundkörper selbsttätig verriegeln. Durch Betätigen eines geeigneten Entriegelungselementes lässt sich ebenfalls ein einfaches Lösen des Substratträgers vom Grundkörper ermöglichen. Alternativ oder in Ergänzung ist es denkbar, bekannte Befestigungselemente, wie etwa Schrauben oder Spannverschlüsse, zu verwenden, um den oder die Substratträger lösbar fest, also insbesondere zerstörungsfrei lösbar, mit dem Grundkörper der Vorrichtung zur Aufzucht von Algen zu verbinden.

Im Weiteren ist es von besonderem Vorteil, wenn der Schwimmkörper der Tarriereinheit einen Hohlkörper aufweist. Vorzugsweise ist mit Hilfe der Stelleinheit zumindest zeitweise wenigstens Medium bedarfsgerecht in den Hohlkörper des Schwimmkörpers einbringbar und aus diesen wieder ablassbar, um hierdurch die Dichte des Schwimmkörpers gezielt zu verändern. In diesem Zusammenhang ist es denkbar, dass die Stelleinheit über eine Pumpe, einen Verdichter und/oder ein Stellorgan, etwa in Form eines Ventils, verfügt, mit dem ein Fluid, beispielsweise Wasser und/oder Umgebungsluft, in den Hohlraum einleitbar und bei Bedarf auch wieder aus dem Hohlraum ausleitbar ist. Wesentlich ist jeweils, dass sich die Dichte des Schwimmkörpers und somit das Verhältnis zwischen der unterhalb der Wasseroberfläche auf die erfindungsgemäße Vorrichtung wirkenden Auftriebskraft und der Masse des Schwimmkörpers bzw. damit auch der Masse der Vorrichtung gezielt verändern lässt. Durch eine Veränderung der Dichte des Schwimmkörpers und des Verhältnisses zwischen auf die Vorrichtung einwirkenden Auftriebs- und Schwerkraft ist es auf vorteilhafte Weise möglich, die erfindungsgemäße Vorrichtung, insbesondere deren Substratträger, in Richtung des Gewässeruntergrundes oder der Gewässeroberfläche zu bewegen oder aber in einer zumindest annähernd gleichbleibenden Tiefe unterhalb der Gewässeroberfläche zu halten

Gemäß einer speziellen Weiterbildung verfügt die Stelleinheit über wenigstens ein Stellorgan, insbesondere Ventil, das bei Erreichen eines vorgegeben Drucks innerhalb des Hohlraums des Schwimmkörpers oder eines vorgegebenen Differenzdrucks zwischen einem Innendruck des Hohlraums und einem Umgebungsdruck öffnet und so die Durchleitung eines Fluids, wie etwa Wasser oder Luft, ermöglicht. Alternativ oder in Ergänzung wird wenigstens ein gezielt ansteuerbares Stellorgan, insbesondere Ventil, verwendet, das aufgrund eines von einer Steuereinheit empfangenen Steuersignals öffnet, schließt oder zumindest zeitweise einen gewünschten Durchfluss eines Fluids sicherstellt. Wesentlich ist stets, dass die Stelleinheit über wenigstens einen geeigneten Aktor verfügt, der die Dichte, hier die Masse des Schwimmkörpers verändert, und zwar derart, dass die auf die erfindungsgemäße Vorrichtung wirkende Schwerkraft die Auftriebskraft übersteigt, sofern die Substratträger abgesenkt werden sollen, und die Auftriebskraft die Schwerkraft übersteigt, sofern der Substratträger in Richtung der Gewässeroberfläche bewegt werden soll. Soll der Substratträger in einer bestimmten Tiefe unterhalb der Wasseroberfläche verbleiben, müssen die Auftriebskraft und die Schwerkraft gleich sein.

In einer ganz speziellen Ausführungsform verfügt der Schwimmkörper über einen Hohlkörper und die Stelleinheit über eine Verdichtereinheit zur Verdichtung von Umgebungsluft und ein Steuerventil, das gezielt geöffnet werden kann und dann einen Strömungskanal zwischen dem Hohlraum und der Umgebung herstellt. Soll in diesem Fall die erfindungsgemäße Vorrichtung, insbesondere der Substratträger, weiter abgesenkt werden, wird das Ventil geöffnet und der Hohlraum zumindest teilweise mit Wasser aus der Umgebung geflutet. Dieser Vorgang kann unterstützt werden, indem die Verdichtereinheit Luft aus dem Hohlraum absaugt. Soll die Vorrichtung nicht weiter abgesenkt werden, wird das Ventil geschlossen und es stellt sich ein Gleichgewicht zwischen Schwerkraft und Auftriebskraft ein. Sofern die Vorrichtung in Richtung der Gewässeroberfläche bewegt werden soll, fördert die Verdichtereinheit verdichtete Umgebungsluft in den Hohlraum und das darin befindliche Wasser wird zumindest teilweise aus dem Hohlraum durch das Ventil in das Gewässer ausgeblasen.

Eine weitere spezielle Ausgestaltung der Erfindung sieht vor, dass zu einer Veränderung der Dichte des Schwimmkörpers dessen Volumen gezielt verändert wird. Dies kann alternativ oder ergänzend zu einer Änderung der Masse des Schwimmkörpers erfolgen. Durch eine Veränderung des Volumens des Schwimmkörpers ändert sich unterhalb der Wasseroberfläche auch das Volumen des verdrängten Wassers und somit der auf die erfindungsgemäße Vorrichtung wirkende statische Auftrieb. Vorzugsweise verfügt die Stelleinheit in diesem Fall über eine Verdichtereinheit, die Umgebungsluft ansaugt und eventuell verdichtet und in ein Element des Schwimmkörpers fördert, das über eine elastische Außenhaut verfügt oder dessen Volumen sich auf sonstige Weise durch das Einblasen von Luft vergrößert. Soll die Dichte des Schwimmkörpers verringert werden, wird Luft über die Verdichtereinheit und/oder ein zusätzlich vorgesehenes, ansteuerbares Ablassventil wieder in die Umgebung abgelassen.

Gemäß einer weiteren Ausführungsform ist die Vegetationsfläche des am Grundkörper befestigten Substratträgers in einem Betriebszustand, bei dem der Substratträger wenigstens teilweisen das Gewässer eingetaucht ist, zumindest nahezu vertikal zur ebenen Wasseroberfläche des Gewässers angeordnet. Ebenso ist es denkbar, dass der Substratträger mit seiner Vegetationsfläche nicht nahezu vertikal angeordnet, aber zumindest schräg gegenüber der Vertikalen ausgerichtet ist. Eine vertikale oder schräge Ausrichtung der Vegetationsflächen ist hierbei vor allem hinsichtlich der Bewegungen bei einer Veränderung der Wassertiefe, in die der Substratträger eingetaucht ist, von Vorteil.

Eine weitere ganz besonders bevorzugte Ausführungsform sieht vor, dass der Grundkörper der Vorrichtung zur Aufzucht von Algen in Form eines Zylinders ausgebildet ist, an dessen Mantelfläche eine Mehrzahl von Substratträgern mit Vegetationsflächen, die vertikal oder schräg zur Mantelfläche des Zylinders angeordnet sind, befestigbar. Eine entsprechend erfindungsgemäß ausgeführte Vorrichtung nimmt hierbei somit die Form eines Propellers an.

Eine weitere spezielle Ausgestaltung der Erfindung zeichnet sich dadurch aus, dass wenigstens ein Sensor vorgesehen ist, mit dem zumindest ein das Wachstum der Algen beeinflussender Parameter im Gewässer erfassbar ist, wobei der Wachstumsparameter ausgewählt ist aus einer Gruppe von Wachstumsparametern, zu denen eine Temperatur, ein Salzgehalt, ein Nährstoffgehalt, ein Nitratgehalt, ein Sauerstoffgehalt, ein Salzgehalt, eine Strömungsgeschwindigkeit und/oder eine Wassertemperatur des Gewässers und/oder eine Intensität, mit der die Gewässeroberfläche mit UV-Strahlung, insbesondere mit Sonnenstrahlung beaufschlagt wird. Mit dieser Weiterbildung der Erfindung wird somit wenigstens ein spezieller Parameter, der das Wachstumsverhalten der zu kultivierenden Algen beeinflusst, erfasst und steht somit für eine vorteilhafte Ansteuerung und sogar Regelung der Tiefe, in der der Substratträger unterhalb der Wasseroberfläche angeordnet wird, zur Verfügung. In diesem Zusammenhang ist es somit denkbar, dass der wenigstens eine erfasste Wachstumsparameter verwendet wird, um auf der Grundlage eines Vergleichs mit einem Sollwert eine gezielte Ansteuerung der Stelleinheit des Schwimmkörpers zu bewirken. Auf diese Weise lässt sich Eintauchtiefe des oder der Substratträger mit den darauf befindlichen Vegetationsflächen und zu kultivierenden Algen bedarfsgerecht an die in einem Gewässer herrschenden Wachstumsbedingungen anpassen. Hierbei ist zu berücksichtigen, dass selbst wenn die Aufzucht generell im gleichen Gewässer erfolgt, Schwankungen der einzelnen Wachstumsparameter aufgrund jahreszeitlicher Änderungen und/oder sich ändern der Wetterbedingungen vorliegen können, genauso wie zum Beispiel der Nährstoff-, Wärme- und Lichtbedarf der zu kultivierenden Pflanzen in Abhängigkeit der Wachstumsphase variieren kann.

Gemäß einer ganz speziellen Ausführungsform der Erfindung ist vorgesehen, dass am Grundkörper wenigstens mittelbar ein Datenlogger, eine Datenspeichereinheit und/oder ein Funkmodul, über das Daten mit Informationen über einen Wachstumszustand der Algen, über eine Temperatur, über einen Sauerstoffgehalt, über einen Salzgehalt, über einen Nährstoffgehalt über einen Nitratgehalt, über eine Strömungsgeschwindigkeit und/oder über eine Oberflächenbestrahlung, insbesondere mit Sonnenlicht, an eine externe Datenverarbeitungs- oder Speichereinheit übertragbar sind, befestigt ist. Mithilfe einer derart erfindungsgemäß ausgeführten Vorrichtung ist somit eine gezielte Fernsteuerung einer entsprechenden Vorrichtung, von denen eine Vielzahl zu einer agrarindustriellen Anlage zur Algenaufzucht zusammengeführt sein können, möglich. Insbesondere der Wachstumsprozess der Algen lässt sich so auf besonders vorteilhafte Weise überwachen und ermöglicht darüber hinaus den gezielten Eingriff, beispielsweise eine Veränderung der Eintauchtiefe der einzelnen Vorrichtungen zur Aufzucht von Algen, auch aus der Ferne.

Im Übrigen betrifft die Erfindung auch ein Verfahren zur Aufzucht von Algen in einem Gewässer, bei dem
- in einem Substrat Samen, Keimlinge, Sämlinge und/oder Setzlinge angeordnet werden und das Substrat auf eine Vegetationsfläche eines Substratträgers aufgebracht wird,
- das auf dem Substratträger angeordnete Substrat wenigstens bereichsweise abgedeckt wird und
- der Substratträger mit dem Substrat an einem Grundkörper befestigt wird.

Das erfindungsgemäße Verfahren zeichnet sich durch folgende Schritte aus:
- Verbinden des Substratträgers wenigstens mittelbar mit einem Schwimmkörper und Absenken des Substratträgers sowie des Schwimmkörpers wenigstens teilweise unter eine Oberfläche des Gewässers sowie
- Einstellen und/oder Verändern einer Dichte des Schwimmkörpers in Abhängigkeit einer Sollwassertiefe, in der sich der am Grundkörper befestigte Substratträger befinden soll.

Mithilfe des erfindungsgemäßen Verfahrens ist es somit möglich, die Eintauchtiefe einer Vorrichtung zur Aufzucht von Algen gezielt zu verändern und so sicherzustellen, dass die Substratträger mit ihren Vegetationsflächen und den darauf angeordneten, zu kultivierenden Algen stets in der Wassertiefe angeordnet sind, die für ein Wachstum der Algen zumindest annähernd optimal ist. Erfindungsgemäß ist hierbei erkannt worden, dass durch ein Einstellen und/oder Verändern der Dichte eines Schwimmkörpers, der wenigstens mittelbar mit einem Grundkörper verbunden ist, an dem die entsprechenden Substratträger befestigt werden, die Eintauchtiefe auf verhältnismäßig einfache Weise verstellbar bzw. auf den gewünschten Wert einstellbar ist. Die Eintauchtiefe wird hierbei in Abhängigkeit der jeweiligen Wachstumsbedingungen in einer bestimmten Tiefe unterhalb der Wasseroberfläche eines Gewässers und/oder der Wachstumsphasen der aufzuziehenden Algen verändert.

Gemäß einer speziellen Weiterbildung des erfindungsgemäßen Verfahrens ist vorgesehen, dass eine Temperatur, ein Sauerstoffgehalt, ein Nährstoffgehalt, ein Nitratgehalt, ein Salzgehalt, eine Strömungsgeschwindigkeit und/oder eine Oberflächenbestrahlung, insbesondere mit Sonnenlicht, des Gewässers erfasst und die Dichte des Schwimmkörpers unter Berücksichtigung wenigstens eines erfassten Wertes eingestellt und/oder verändert wird. Gemäß dieser speziellen Ausführungsform der Erfindung wird somit eine bedarfsgerechte Regelung der Eintauchtiefe der Substratträger mit den Vegetationsflächen und den darauf angeordneten zu kultivierenden Algen erreicht. Die Substratträger können so stets in Abhängigkeit des oder der erfassten Werte tiefer in das Gewässer eingetaucht oder aber in Richtung der Wasseroberfläche bewegt werden

Im Weiteren wird die Erfindung ohne Beschränkung des allgemeinen Erfindungsgedankens anhand spezieller Ausführungsbeispiele unter Bezugnahme auf die Figuren näher erläutert. Dabei zeigen:
- Fig. 1:: Darstellung einer erfindungsgemäß ausgeführten Vorrichtung zur Aufzucht von Algen;
- Fig. 2:: Detailansichten und Zusammenbaudarstellung eines Substratträgers einer erfindungsgemäß ausgeführten Vorrichtung zur Aufzucht von Algen
- Fig. 3:: Darstellung einer in einem Gewässer angeordneten, erfindungsgemäß ausgeführten Vorrichtung zur Aufzucht von Algen;
- Fig. 4:: Darstellung einer speziellen konstruktiven Ausgestaltung einer erfindungsgemäß ausgeführten Vorrichtung zur Aufzucht von Algen;
- Fig. 5:: Anordnung mit einer Mehrzahl von erfindungsgemäß ausgeführten Vorrichtungen zur Aufzucht von Algen in einem Gewässer.

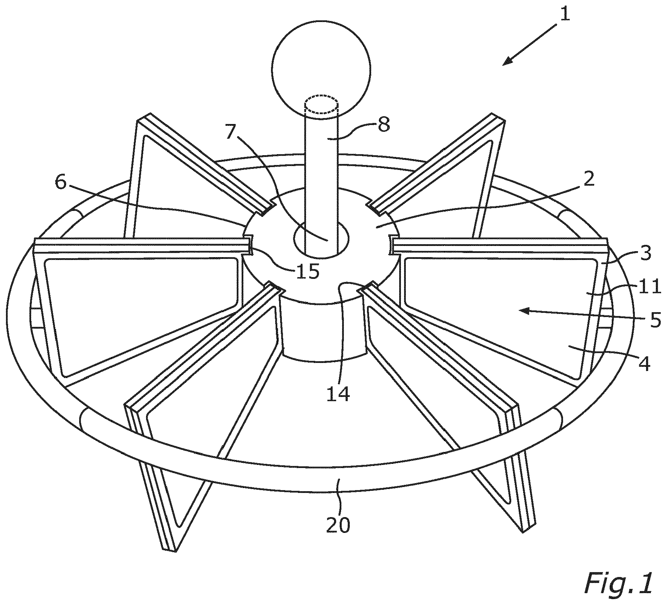

Fig. 1 zeigt eine spezielle Ausführungsform der erfindungsgemäß ausgeführten Vorrichtung 1 zur Aufzucht von Algen. Die Vorrichtung 1 verfügt über einen Grundkörper 2 der zylinderförmig ausgebildet ist und an dessen Mantelfläche eine Mehrzahl von Substratträgern 3 befestigt ist, wobei die einzelnen Substratträger 3 Befestigungselemente 15 in Form von Schienen aufweisen, die in auf der Mantelfläche angeordneten, als Aufnahmen 14 dienende Nuten eingeschoben sind. Zur weiteren Fixierung der Substratträger 3 ist am äußeren Umfang der nach außen gerichteten Substratträger 3 ein ringförmiger ein Rahmen 20 vorgesehen, der die einzelnen Substratträger 3 miteinander verbindet und so die Stabilität der gesamten Vorrichtung 1 erhöht. Auch der ringförmige Rahmen 20 lässt sich bei Bedarf werkzeuglos entfernen.

Im Weiteren verfügt die Vorrichtung 1 über eine Tarriereinheit 6 mit einer Stelleinheit 8 und einem Schwimmkörper 7, die eingerichtet ist, um eine gezielte Bewegung der Vorrichtung 1, insbesondere der Substratträger 3, in eine Sollwassertiefe, also eine gezielte Bewegung in Richtung des Gewässergrundes oder aber in Richtung der Gewässeroberfläche 9 zu erreichen. Wesentlich für die Vorrichtung ist, dass diese gezielte Bewegung oder das Halten der Vorrichtung 1 in einer bestimmten Tiefe, in Abhängigkeit der in einem Gewässer 10 herrschenden Wachstumsbedingen für die zu kultivierenden Algen und/oder der Wachstumsphasen der Algen erfolgt, sodass die Kultivierung der Algen unter zumindest nahezu optimale Wachstumsbedingungen erfolgt.

Wesentlich für die Tarriereinheit 6 ist, dass die Dichte des Schwimmkörpers 7 mithilfe der Stelleinheit 8 veränderbar ist, sodass die Vorrichtung 1 zur Aufzucht von Algen gezielt entweder in eine gewünschte Wassertiefe bewegbar oder aber in dieser haltbar ist. Generell ist es hierbei denkbar, die Dichte des Schwimmkörpers 7 durch Veränderung seiner Masse und/oder seines Volumens zu verändern.

Die einzelnen Substratträger 3 sind austauschbar an dem Grundkörper 2 befestigt, wobei gemäß dem in Fig. 1 dargestellten Ausführungsbeispiel der Grundkörper 2 an seiner Mantel- oder Zylinderaußenfläche in Längsrichtung verlaufenden Nuten als Aufnahmen 14 aufweist, in die entsprechende an den Substratträgern 3 angeordnete Nutsteine oder Schienen, die als Befestigungselemente 15 dienen, einführbar sind. Gemäß dem beschriebenen Ausführungsbeispiel sind die Substratträger 3 während des Aufenthalts in einem Gewässer 10 mit ihrer Haupterstreckungsrichtung zumindest nahezu vertikal zur Wasseroberfläche 9 angeordnet.

Ferner verfügen die einzelnen Substratträger 3 auf zumindest einer Seite über eine Vegetationsfläche 4 auf die ein Substrat 5, in dem sich Samen, Keimlinge, Sämlinge und/oder Setzlinge befinden, aufgebracht ist. Eine Fixierung des Substrats 5 auf der Vegetationsfläche 4 erfolgt hierbei mithilfe eines Abdeckelements 11, das am Substratträger 3 befestigbar ist und die Vegetationsfläche 4 mit dem darauf angeordneten Substrat 5 überragt. Auf diese Weise ist das Substrat 5 mit den Samen, Keimlingen, Sämlinge und/oder Setzlingen in einem Zwischenraum 13 zwischen der Vegetationsfläche 4 des Substratträgers 3 und dem Abdeckelement 11, bei dem es sich vorzugsweise um ein Gitterelement oder eine Lochplatte handelt, angeordnet und fixiert.

Sobald die in Fig. 1 gezeigte Vorrichtung 1 mit dem auf den einzelnen Vegetationsflächen 4 der Substratträger 3 angeordneten Substrat 5 in das zur Aufzucht von Algen ausgewählte Gewässer 10 eingetaucht wird, dringt Wasser durch die Öffnungen 12 des Abdeckelements 11 hindurch und versorgt die zu kultivierenden Algen in ihrer jeweiligen Wachstumsphase mit Wasser und Nährstoffen.

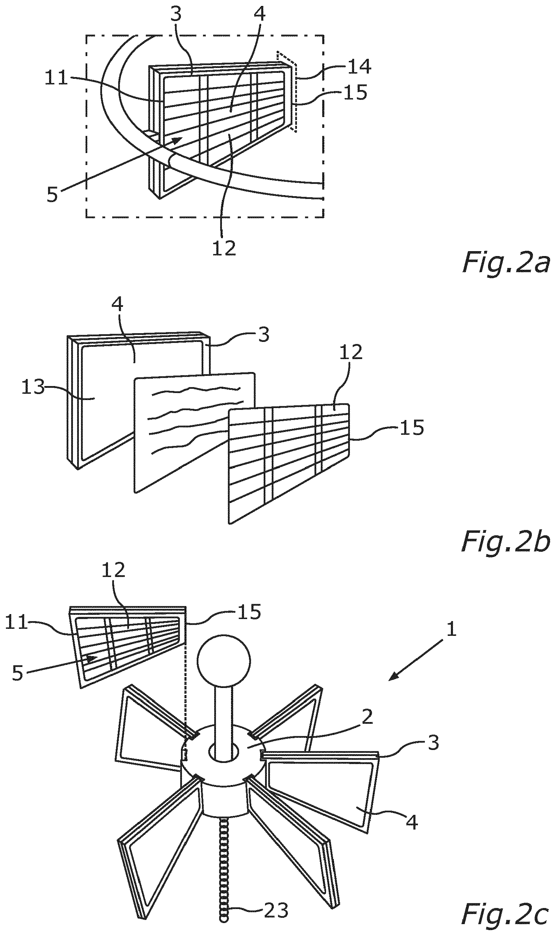

In Ergänzung zu Fig. 1 zeigt Fig. 2 einen einzelnen Substratträger 3 einer erfindungsgemäßen Vorrichtung 1 in zwei Detailansichten sowie einer Zusammenbaudarstellung. In Fig. 2 a) ist der Substratträger 3 nochmals in einem Betriebszustand gezeigt, in dem dieser an dem Grundkörper 2 befestigt ist und sich zwischen dem Grundkörper 2 und einem den Substratträger 3 an der äußeren Kante haltenden Rahmen 20 befindet. Fig. 2 b) zeigt im Übrigen die einzelnen Komponenten eines Substratträgers 3, nämlich die Vegetationsfläche 4 zur Anordnung eines Substrats 5 und ein an dem Substratträger 3 befestigbares Abdeckelement 11, sowie das Substrat 5, in dem zur Aufzucht von Algen geeignete Samen, Keimlinge, Sämlinge und/oder Setzlinge angeordnet sind. Die Ausgestaltung des Abdeckelements 11 kann hierbei in Abhängigkeit der Art der zu kultivierenden Algen und/oder jeweiligen Wachstumsphase bedarfsgerecht ausgebildet bzw. angepasst sein. Vorzugsweise ist das Abdeckelement 11 entweder als Gitterelement, wie in Figur 2 b) dargestellt, oder aber als Lochplatte ausgeführt. Ergänzend zeigt Fig. 2 c) einen Montagezustand einer erfindungsgemäß ausgeführten Vorrichtung 1, bei dem bereits fünf Substratträger 3 am Grundkörper 2 befestigt wurden und gerade der sechste Substratträger 3 mit seinen Befestigungselementen 15 in den Aufnahmen 16 an der Mantelfläche des zylinderförmigen Grundkörpers 2 fixiert werden soll. Gemäß der dargestellten Ausführungsform ist die erfindungsgemäße Vorrichtung 1 über eine Verankerung mit einem Verbindungselement, hier in Form einer Ankerkette 23, mit dem Gewässeruntergrund verbunden.

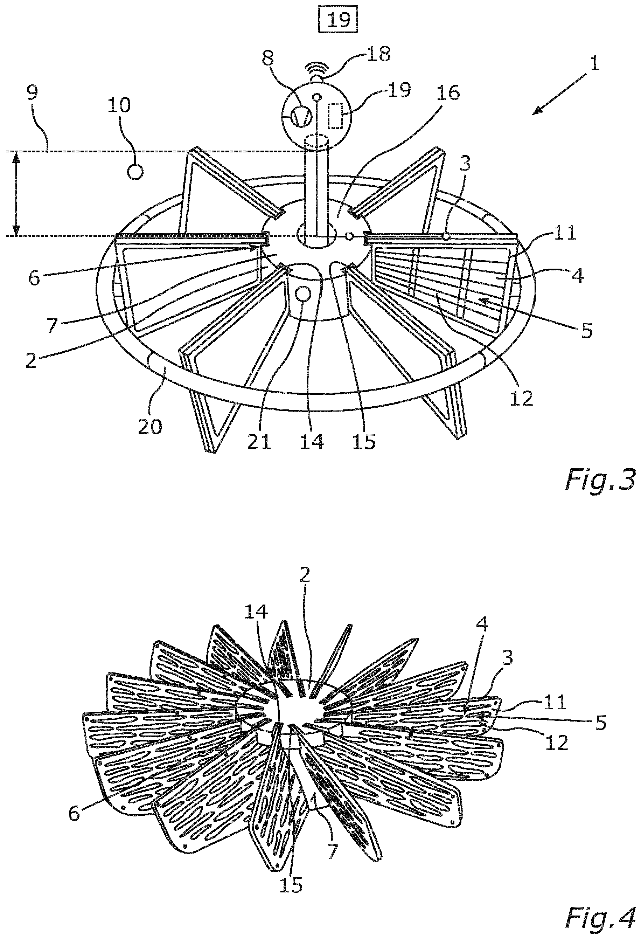

Fig. 3 zeigt die erfindungsgemäß ausgeführte Vorrichtung 1 in einem Betriebszustand, in dem diese sich zumindest teilweise unterhalb der Wasseroberfläche 9 eines Gewässers 10 befindet. Hierbei wurde die Vorrichtung 1 durch geeignete Ansteuerung der Stelleinheit 8 der Tarriereinheit 6 in die erforderliche Wassertiefe abgesenkt. Aufgrund der erfindungsgemäßen Ausgestaltung der Vorrichtung 1 zur Aufzucht von Algen erfolgt in dem beschriebenen Fall das Absenken der Vorrichtung 1 mit ihren Substratträgern 3 und den darauf angeordneten zu kultivierenden Algen in die optimale Wassertiefe automatisiert. Um einen entsprechend automatisierten bzw. geregelten Prozess zur bedarfsgerechten Verbringung und/oder Arretierung der Vorrichtung 1 in unterschiedlichen Wassertiefen zu erreichen, verfügt die in Fig. 3 dargestellte Vorrichtung 1 über Sensoren 17, mit denen der Salzgehalt, der Nitratgehalt, die Temperatur das Wachstumsverhalten der Algen sowie die auf die Oberfläche 9 des Gewässers 10 einwirkende Sonnenstrahlung gemessen wird. Die einzelnen vorgenannten Parameter werden erfasst und einer Datenauswerteeinheit 19 zugeführt, die unter Berücksichtigung der einzelnen Messwerte die optimale, für die jeweilige Wachstumsphase der zu kultivierenden Algen optimale Eintauchtiefe ermittelt und diese als Solltiefe dem Einstellungsprozess zugrunde legt. Die Datenverarbeitungseinheit 19 kann hierbei Teil der Vorrichtung 1 sein oder aber als externe Datenverarbeitungseinheit 19 ausgeführt sein, die über ein an der Vorrichtung befestigtes Funkmodul 18 bidirektional Signale und/oder Daten austauscht.

Gemäß der Fig. 3 dargestellten Ausführungsform erfolgt die Veränderung der Eintauchtiefe der Vorrichtung 1 mit ihren Substratträgern 3 und den darauf angeordneten aufzuziehenden Algen, sobald in der Datenverarbeitungseinheit 19 eine Differenz zwischen der Solltiefe und der Isttiefe detektiert wird. Um in einem solchen Fall eine gezielte Verstellung der Eintauchtiefe vornehmen zu können, ist eine Tarriereinheit 6 mit einer Stelleinheit 8 und einem Schwimmkörper 7 vorgesehen.

Die Stelleinheit 8 ist gemäß der beschriebenen Ausführungsform derart ausgebildet, dass die die Dichte des Schwimmkörpers 7 durch eine Veränderung seiner Masse gezielt in Abhängigkeit der erfassten Messwerte verändert wird. Ebenso ist es möglich, nach Abschluss des gewünschten Wachstumsprozesses der Algen die Vorrichtung 1 gesteuert an die Wasseroberfläche 9 zu holen, um eine Ernte der Algen durchführen zu können.

Gemäß der in Fig. 3 dargestellten Ausführungsform verfügt die Stelleinheit 8 über eine Verdichtereinheit, mit der Umgebungsluft oberhalb der Wasseroberfläche 9 angesaugt, verdichtet und in einen Hohlraum 16 des Schwimmkörpers 7 gefördert werden kann. Weiterhin verfügt die Stelleinheit 8 über ein Stellorgan 21, bevorzugt in Form eines Ventils, das sich auf einen Steuerbefehl hin öffnen lässt, sodass Wasser aus dem Gewässer 10 in den Hohlraum 16 einströmen kann. In geschlossenem Zustand verschließt dieses Ventil hingegen den Hohlraum 16 flüssigkeitsdicht gegenüber dem umgebenden Gewässer 10. Über ein weiteres Ventil oder die Verdichtereinheit kann im Übrigen bei Bedarf überschüssige Luft aus dem Hohlraum 16 entweichen.

Sobald die erfindungsgemäße Vorrichtung 1 gemäß Fig. 3 weiter abgesenkt werden soll, wird das zuvor beschriebene Ventil geöffnet und Wasser strömt in dem gewünschten Maß in den Hohlraum 16 des Schwimmkörpers 7 ein, sodass sich dessen Dichte erhöht und dieser absinkt. Soll dieser Vorgang gestoppt werden, wird das Ventil geschlossen und die Vorrichtung 1 verbleibt mit den zu kultivierenden Algen in der gewünschten Wassertiefe unterhalb der Gewässeroberfläche 9. Soll dagegen eine Bewegung in Richtung der Gewässeroberfläche 9 initiiert werden, saugt die Verdichtereinheit Umgebungsluft an, verdichtet diese und bläst das im Hohlraum 16 vorhandene Wasser so lange über ein steuerbares Ventil oder ein Überdruckventil aus, bis sich die Vorrichtung 1 in der gewünschten Wassertiefe befindet. Befindet sich im Hohlraum 16 des Schwimmkörpers 7 kein Wasser, so schwimmt die erfindungsgemäße Vorrichtung 1 an der Wasseroberfläche 9, sodass etwa Ernte-, Wartungs- oder Reparaturarbeiten durchgeführt werden können.

Fig. 4 zeigt eine ganz besonders spezielle Ausgestaltung einer erfindungsgemäß ausgeführten Vorrichtung 1 zur Aufzucht von Algen. Die Vorrichtung 1 verfügt wiederum über einen Grundkörper 2, an dem umfangseitig eine Mehrzahl von speziell ausgebildeten Substratträgern 3 angeordnet ist. Die Substratträger 3 verfügen jeweils über eine Vegetationsfläche 4, auf der ein Substrat 5 angeordnet werden kann, sowie ein Abdeckelement 11 mit verschiedenen Öffnungen 12, durch die in getauchtem Zustand der Substratträger 3 Wasser in Richtung der Vegetationsflächen 4 der Substratträger mit dem darauf angeordneten Substrat 5 einströmen kann. Die einzelnen Abdeckelemente 11 sind lösbar an den Substratträgern 3 befestigt.

Im Weiteren ist von Bedeutung, dass sowohl die Substratträger 3 als auch die Abdeckelement 11 aus Holz gefertigt sind, sodass eine besonders umweltverträgliche Aufzucht von Algen in Gewässern 10 ermöglicht wird. Die einzelnen Substratträger 3 sind lösbar an dem Grundkörper 2 befestigt, der im Übrigen über eine Tarriereinheit 6 verfügt, die, wie zuvor beschrieben, eine gezielte Bewegung der Vorrichtung 1 in die gewünschte bzw. benötigte Eintauchtiefe unterhalb einer Gewässeroberfläche 9 gewährleistet. Nach Abschluss der Wachstumsphase wird die gesamte Vorrichtung 1 an die Gewässeroberfläche geholt, wo der Erntevorgang durchgeführt werden kann. Ist der Erntevorgang beendet, können die Vegetationsflächen 4 der einzelnen Substratträger 3 gereinigt und wieder mit Substrat 5, in dem sich Samen, Keimlinge, Sämlinge und/oder Setzlinge befinden, beaufschlagt sowie mit den Abdeckelementen 11 abgedeckt werden. Daraufhin werden die einzelnen Substratträger 3 erneut am Grundkörper 2 befestigt und die Vorrichtung 1 steht zur Realisierung einer weiteren Wachstumsphase für Algen zur Verfügung.

Mit Hilfe der erfindungsgemäßen technischen Lösung wird somit eine besonderes effiziente Algenaufzucht ermöglicht, bei der einerseits besonders umweltverträgliche Materialien zum Einsatz kommen und andererseits eine bedarfsgerechte Einstellung der Tauchtiefe in Abhängigkeit der benötigten Wachstumsparameter und der Wachstumsphasen der zu kultivierenden Algen erreicht wird. Auf diese Weise lässt sich nicht nur eine umweltverträgliche, sondern auch eine besonders effiziente Algenaufzucht umsetzen.

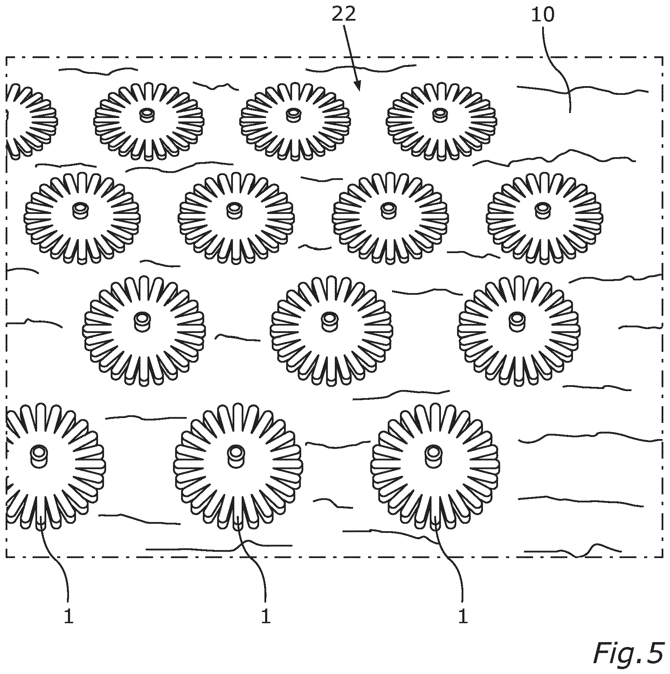

Abschließend zeigt Fig. 5 eine Anordnung 22 mit einer Vielzahl von erfindungsgemäß ausgeführten Vorrichtungen 1, die in einem Gewässer 10, etwa einer küstennahen Meeresbucht, angeordnet sind. Der einzelnen erfindungsgemäß ausgeführten Vorrichtungen 1 sind auf vorteilhafte Weise miteinander verbunden und am Meeresgrund verankert, so dass diese einerseits einen festen Verbund bilden, andererseits aber unter Berücksichtigung unterschiedlicher Wachstumsbedingungen einzeln, in Teilgruppen oder gemeinsam in unterschiedliche Wassertiefen bewegt werden können. Mithilfe der in Fig. 5 gezeigten Anordnung 22, die eine Vielzahl von erfindungsgemäß ausgeführten Vorrichtungen 1 zur Aufzucht von Algen aufweist, kann eine besonders umweltverträgliche und darüber hinaus effiziente Algenaufzucht erreicht werden, insbesondere, wenn die Anlage fernüberwachbar und/oder fernsteuerbar ist.

### Bezugszeichenliste

- 1: Vorrichtung zur Aufzucht von Algen
- 2: Grundkörper
- 3: Substratträger
- 4: Vegetationsfläche
- 5: Substrat
- 6: Tarriereinheit
- 7: Schwimmkörper
- 8: Stelleinheit
- 9: Wasseroberfläche
- 10: Gewässer
- 11: Abdeckelement
- 12: Öffnung
- 13: Zwischenraum
- 14: Aufnahme
- 15: Befestigungselement
- 16: Hohlraum
- 17: Sensor
- 18: Funkmodul
- 19: Datenverarbeitungseinheit
- 20: Rahmen
- 21: Stellorgan
- 22: Anordnung mehrerer Vorrichtungen
- 23: Ankerkette

## Patentansprüche

1. Vorrichtung (1) zur Aufzucht von Algen in einem Gewässer (10) mit einem Grundkörper (2), an dem ein Substratträger (3) mit einer Vegetationsfläche (4) zur Aufbringung eines Substrats (5), in dem sich Samen, Keimlinge, Sämlinge und/oder Setzlinge befinden, befestigbar oder befestigt ist, wobei der Substratträger (3) mit der Vegetationsfläche (4) zumindest zeitweise unter eine Wasseroberfläche (9) des Gewässers (10) absenkbar ist,
**dadurch gekennzeichnet, dass** eine Tarriereinheit (6) vorgesehen ist, die wenigstens mittelbar mit dem Substratträger (3) verbunden oder verbindbar ist und die über einen Schwimmkörper (7) und über eine Stelleinheit (8), durch die eine Dichte des Schwimmkörpers (7) veränderbar ist, verfügt.

2. Vorrichtung nach Anspruch 1,
**dadurch gekennzeichnet, dass** die Tarriereinheit (6) Teil des Grundkörpers (2) ist oder den Grundkörper (2) bildet.

3. Vorrichtung nach Anspruch 1 oder 2,
**dadurch gekennzeichnet, dass** die Vegetationsfläche (4) des Substratträgers (3) unterhalb eines Abdeckelements (11) angeordnet ist, das eine Mehrzahl von Öffnungen (12) aufweist, durch die Wasser in einen zwischen dem Abdeckelement (11) und der Vegetationsfläche (4) angeordneten Zwischenraum (13) leitbar ist.

4. Vorrichtung nach Anspruch 3,
dass das Abdeckelement (11) eine Perforation, ein Gitter und/oder eine Lochplatte aufweist.

5. Vorrichtung nach einem der vorangehenden Ansprüche,
**dadurch gekennzeichnet, dass** der Substratträger (3) lösbar fest und/oder auswechselbar am Grundkörper befestigbar ist.

6. Vorrichtung nach einem der vorangehenden Ansprüche,
**dadurch gekennzeichnet, dass** auf der Vegetationsfläche (4) wenigstens bereichsweise ein Schwammmaterial angeordnet ist und/oder das Substrat (5) wenigstens teilweise ein Schwammmaterial aufweist.

7. Vorrichtung nach einem der vorangehenden Ansprüche,
**dadurch gekennzeichnet, dass** sich auf der Vegetationsfläche (4) ein Wachstumssubstrat mit wenigstens einem Nährstoff befindet, in dem die Samen, Keimlingen, Sämlingen und/oder Setzlingen angeordnet sind.

8. Vorrichtung nach einem der vorangehenden Ansprüche,
**dadurch gekennzeichnet, dass** an dem Grundkörper (2) eine Aufnahme (14) zur zerstörungsfreien Befestigung eines Substratträgers (3) und zum zerstörungsfreien Lösen eines befestigten Substratträgers (3) angeordnet ist und/oder, dass der Substratträger (3) ein Befestigungselement (15) aufweist, das in die Aufnahme (14) einführbar, einsteckbar, einschraubbar und/oder einrastbar ist.

9. Vorrichtung nach einem der vorangehenden Ansprüche,
**dadurch gekennzeichnet, dass** der Schwimmkörper (7) einen Hohlraum (16) und/oder eine elastisch verformbare Oberfläche aufweist.

10. Vorrichtung nach einem der vorangehenden Ansprüche,
**dadurch gekennzeichnet, dass** die Stelleinheit (8) eine Pumpe, einen Verdichter zur Förderung eines Fluids in den Schwimmkörper (7) und/oder einen Aktor, durch den ein Volumen des Schwimmkörpers (7) veränderbar ist, aufweist.

11. Vorrichtung nach einem der vorangehenden Ansprüche,
**dadurch gekennzeichnet, dass** die Vegetationsfläche (4) des am Grundkörper (2) befestigten Substratträgers (3) in einem Betriebszustand, bei dem der Substratträger (3) wenigstens teilweise in das Gewässer (10) eingetaucht ist, zumindest nahezu vertikal zur ebenen Wasseroberfläche (9) des Gewässers (10) angeordnet ist.

12. Vorrichtung nach einem der vorangehenden Ansprüche,
**dadurch gekennzeichnet, dass** ein Sensor (17) vorgesehen ist, mit dem wenigsten ein Wachstumsparameter im oder am Gewässer (10) erfassbar ist, wobei der Wachstumsparameter ausgewählt ist aus einer Gruppe von Wachstumsparametern, zu denen eine Wassertemperatur, ein Salzgehalt, ein Nährstoffgehalt, ein Nitratgehalt, ein Sauerstoffgehalt, ein Wert der in das Gewässer eingestrahlten Strahlung.

13. Vorrichtung nach einem der vorangehenden Ansprüche,
**dadurch gekennzeichnet, dass** am Grundkörper (2) wenigstens mittelbar ein Datenlogger, eine Datenspeichereinheit und/oder ein Funkmodul (18), über das Daten mit Informationen über einen Wachstumszustand der Algen, über eine Temperatur, über einen Sauerstoffgehalt, über einen Nährstoffgehalt, über eine Strömungsgeschwindigkeit und/oder über eine Oberflächenbestrahlung, insbesondere mit Sonnenlicht, der Oberfläche des Gewässers an eine externe Datenverarbeitungs- oder -speichereinheit (19) übertragbar sind, befestigt ist.

14. Verfahren zur Aufzucht von Algen in einem Gewässer (10), bei dem
- in einem Substrat (5) Samen, Keimlinge, Sämlinge und/oder Setzlinge angeordnet werden und das Substrat (5) auf eine Vegetationsfläche (4) eines Substratträgers (3) aufgebracht wird,
- das zumindest mittelbar auf dem Substratträger (3) angeordnete Substrat (5) wenigstens bereichsweise abgedeckt wird und
- der Substratträger (3) mit dem Substrat (5) an einem Grundkörper (2) befestigt wird,
**gekennzeichnet durch** die Schritte,
- Verbinden des Substratträgers (3) wenigstens mittelbar mit einem Schwimmkörper (7) und Absenken des Substratträgers (3) sowie des Schwimmkörpers (7) wenigstens teilweise unter eine Oberfläche (9) des Gewässers (10) sowie
- Einstellen und/oder Verändern einer Dichte des Schwimmkörpers (7) in Abhängigkeit einer Sollwassertiefe, in der sich der am Grundkörper (2) befestigte Substratträger (3) befinden soll.

15. Verfahren nach Anspruch 14,
**dadurch gekennzeichnet, dass** eine Temperatur, ein Sauerstoffgehalt, ein Nährstoffgehalt, eine Strömungsgeschwindigkeit und/oder eine Oberflächenbestrahlung, insbesondere mit Sonnenlicht, der Oberfläche des Gewässers erfasst und die Dichte unter Berücksichtigung wenigstens eines erfassten Werts eingestellt und/oder verändert wird.
